Europäisches Patentamt

European Patent Office

Office européen des brevets

(19)

(11) Veröffentlichungsnummer: **0 013 949**
**B1**

(12) **EUROPÄISCHE PATENTSCHRIFT**

(45) Veröffentlichungstag der Patentschrift: **29.06.83**

(51) Int. Cl.³: **A 61 K 9/22**, A 61 K 9/06

(21) Anmeldenummer: **80100236.1**

(22) Anmeldetag: **18.01.80**

(54) Depotkörper auf Basis Silicon-Kautschuk sowie Verfahren zu seiner Herstellung.

(30) Priorität: **23.01.79 DE 2902414**

(43) Veröffentlichungstag der Anmeldung:
**06.08.80 Patentblatt 80/16**

(45) Bekanntmachung des Hinweises auf die Patenterteilung:
**29.06.83 Patentblatt 83/26**

(84) Benannte Vertragsstaaten:
**AT BE CH DE FR GB IT NL SE**

(56) Entgegenhaltungen:
**DE - B - 2 125 464**
**FR - A - 2 150 851**
**FR - A - 2 309 212**
**US - A - 3 279 996**
**US - A - 3 946 106**

**CHEMICAL ABSTRACTS, Band 90, Nr. 8, 19. Februar 1979, Seite 385, Nr. 61156k Columbus, Ohio, U.S.A. J. W. McGINITY et al.: "Formulation factors influencing the in vitro release of morphine sulfate from silastic pellets"**

(73) Patentinhaber: **HOECHST AKTIENGESELLSCHAFT**
**Postfach 80 03 20**
**D-6230 Frankfurt/Main 80 (DE)**

(72) Erfinder: **Reul, Bernhard**
**Bahnstrasse 25**
**D-6240 Königstein Taunus (DE)**
Erfinder: **Hiller, Dietrich, Dr.**
**Riederbergstrasse 11**
**D-6200 Wiesbaden (DE)**

## 0 013 949

### Depotkörper auf Basis Silicon-Kautschuk sowie Verfahren zu seiner Herstellung

Die vorliegende Erfindung betrifft Depotkörper, insbesondere zur Behandlung von Rindern, sowie die Verfahren zu ihrer Herstellung. Die Depotkörper sind zur Applikation von Wirkstoffen auf der Haut, zum Beispiel der Nasenschleimhaut, von Rindern geeignet.

Der vorliegenden Erfindung liegt die Aufgabe zugrunde, wirkstoffhaltige Depotkörper zur Verfügung zu stellen, welche zur Verabreichung von Wirkstoffen über einen längeren Zeitraum geeignet sind. Die Depotkörper sollen sich insbesondere zur Applikation von Wirkstoffen mit Hilfe von Vorrichtungen, wie sie z.B. in der DE—PS 2 125 464 beschrieben sind, eignen. An diese Applikationsart werden besonders hohe Anforderungen gestellt:

Der oder die Wirkstoffe, ihre Dosierung und die Verabreichungsdauer liegen aus den jeweiligen medizinischen Gründen fest. Die bei einer Applikation auf die Schleimhaut des Nasenvorhofs zwangsläufig auftretenden Wirkstoff-Verluste müssen durch eine höhere Wirkstoffmenge ausgeglichen werden, was infolge des am Applikationsort begrenzten Raumes eine hohe Wirkstoff-Konzentration im Depotkörper bedingt. Die Freigabe der Wirkstoffe wird durch ihre Eigenschaften und die des Trägermaterials bestimmt. Die Wirkstoff-Freigabe soll über lange Zeit, zum Beispiel 6 Wochen bis 3 Monate, möglichst hoch und genau kontrollierbar erfolgen. Tragezeiten von Wochen bis Monaten stellen besonders hohe Ansprüche an die pharmakologische Unbedenklichkeit der verwendeten Stoffe sowie an die Reizlosigkeit der eingesetzten Materialien. Die Arzneiformen müssen während dieser Zeit, bedingt durch die Freßgewohnheiten der Tiere, gegen das Nasensekret und die Bestandteile der Nahrung, zum Beispiel bei Kälbern gegen die Milchnahrung, beständig bleiben. Erforderlich ist eine Beständigkeit auch gegen die mechanische Beanspruchung, der die Depotkörper während einer so langen Tragezeit in der Nase des Tieres ausgesetzt sind. Die Arzneiform muß unter Berücksichtigung aller Gesichtspunkte haltbar und rationell herstellbar sein.

Es wurde nun gefunden, daß die Aufgabe durch die Bereitstellung eines Depotkörpers auf Basis Silicon-Kautschuk gelöst werden kann. Der Depotkörper ist dadurch gekennzeichnet, daß er neben dem oder den Wirkstoffe(n) Silicon-Kautschuk und gegebenenfalls festen Zuschlagstoffen 2 bis 50 Gew.% bezogen auf Silicon-Kautschuk einer freigabefördernden Substanz ausgewählt aus der Gruppe bestehen aus Alkohol, Ester, Äther und Keton mit einer Anzahl von 8 bis 60 C-atomen einzeln oder im Gemisch in gelöster Form enthält.

Die erfindungsgemäßen Depotkörper geben die Wirkstoffe in vorbestimmter Menge pro Zeiteinheit frei. Sie bleiben während der Applikationszeit praktisch formstabil. Das Trägermaterial ist auf Basis von physiologisch akzeptablem Silicon-Kautschuk aufgebaut. Er muß zum Beispiel bestimmte Reinheitskriterien erfüllen. Die Auswahl der Hilfsstoffe sowie Gestalt und Oberfläche des Depotkörpers berücksichtigen die anatomischen Verhältnisse am Applikationsort, die biopharmazeutischen Anforderungen und die Möglichkeit einer rationellen technischen Realisierung.

Die Depotkörper eignen sich insbesondere zur Applikation von Wirkstoffen mit Hilfe von Vorrichtungen, wie sie z.B. in der DE—PS 2 125 464 beschrieben sind. Solche Vorrichtungen werden im folgenden als Nasenspange bezeichnet und bestehen vorzugsweise aus einem Bügel und zwei Halterungen für je einen Depotkörper.

Neben der Applikation von Wirkstoffen auf der Haut sind auch andere Anwendungsarten der erfindungsgemäßen Depotkörper möglich, wie zum Beispiel die Implantation. Die Anwendung der erfindungsgemäßen Zusammensetzung kann auch durch Einspritzen der noch flüssigen Masse in ein Gewebe erfolgen, woran sich dort die Vernetzung und damit die Ausbildung des Depotkörpers anschließt.

Silicon-Kautschuk zeichnet sich durch chemische Indifferenz, Beständigkeit, hohe Permeabilität und außerdem durch liechte Verarbeitbarkeit aus. Die Eigenschaften des Silicon-Kautschuks, wie geringe Schrumpfung und niedriger Ausdehungskoeffizient, begünstigt die Befestigung der Depotkörper auf Vorrichtungen zur Applikation.

Die Verwendung von Silicon-Kautschuk als Träger für Wirkstoffe und zur Beeinflussung der Wirkstoff-Freigabe ist bekannt. Der verwendete Silicon-Kautschuk wird bevorzugt durch Peroxide, Zinn- oder Platin-Verbindungen vulkanisiert. Solche Arneiformen sind zur Implantation oder Einführung in Körperhöhlen bestimmt. Die Wirkstoffe sind im Silicon-Kautschuk gelöst oder suspendiert. Die Arzneiformen haben Matrix Typ oder liegen als Membran-Matrix-Typ vor (vgl. z.B. P. J. Dziuk und B. Cook, Endocrinology *78*, 208 (1966) und US—PS 3 279 996).

Eine besondere Ausführungsform ist die Matrix aus Silicon-Kautschuk mit emulgiertem hydrophilen wirkstoffhaltigen Lösungssystemen, gegebenenfalls umhüllt mit einer Membran, wie sie in DE—OS 2 547 378 beschrieben ist.

Es wurde auch schon beschrieben, daß die Morphinsulfatfreigabe aus Pellets auf Basis Silicon-Kautschuk durch Zusatz von Lactose, Natriumalginat oder Natriumlaurylsulfat verbessert werden kann (Expo.-Congr. Int. Technol. Pharm., 1st. 1977, 2, 235—42). Diese die Freigabefördernden Substanzen sind wasserlöslich.

Den beschriebenen Arzneiformen auf Basis Silicon-Kautschuk fehlt der Zusatz eines Diffusionsförderers mit den oben angegebenen Charakteristika.

2

Wegen der hohen Anforderungen, welche an den Depotkörper gestellt werden, befriedigten solche auf Basis der beschriebenen Vorschläge nicht.

Wider Erwarten wurden unter den schwer wasserlöslichen, im Silicon-Kautschuk löslichen Zusätzen solche gefunden, die in Konzentrationen von 2—50% bezogen auf Silicon-Kautschuk eine befriedigende Wirkstoff-Freigabe aus dem Depotkörper bewirken. Darüber hinaus beeinflussen diese Stoffe die mechanischen Eigenschaften des Silicon-Kautschuks nicht oder nicht wesentlich. Diese Zusätze vermindern die Viskosität der Silicon-Masse und erlauben damit höhere Wirkstoff-Konzentrationen. Der Zusatz dieser Stoffe ist Voraussetzung für die Herstellung von Depotkörpern, welche die oben geschilderten hohen Anforderungen erfüllen, so daß auch therapeutisch wirksame Konzentrationen im Tier erreicht werden.

Als Silicon-Kautschuk eignen sich bevorzugt solche auf Basis von durch Addition oder Kondensation vernetzen Ein- oder Zweikomponenten-Systemen. Silicon-Kautschuke auf Basis Dimethylpolysiloxan (wie ®Silgel 601 der Fa. Wacker Chemie GmbH, eine additionsvernetzende 2-Komponenten-Masse aus 9 Teilen Komponente A und 1 Teil Komponente B), Dimethyl-diphenylpolysiloxan, Dimethylpolysiloxanol und Silicon-Copolymeren sind besonders geeignet.

Die freigabefördernden physiologisch verträglichen im Silicon-Kautschuk löslichen Zusatzstoffe sind lipoidlösliche, schwer wasserlösliche Alkohole, Ester, Äther und Ketone mit einer Anzahl von 8 bis 60 Kohlenstoffatomen. Der Depotkörper kann einen oder mehrere dieser Zusatzstoffe, also ein Gemisch dieser freigabefördernden Substanzen, enthalten.

Die Freigabe des Wirkstoffs bzw. der Wirkstoffe hängt unter anderem ab von der Konzentration der freigabefördernden Substanz bzw. des freigabefördernden Gemisches. Sie ist um so größer, je mehr freigabefördernde Substanz in dem Silicon-Kautschuk gelöst ist. Als freigabefördernde Substanzen werden daher solche eingesetzt, die eine ausreichend hohe Löslichkeit in der Silionmasse besitzen. Ein weiteres wesentliches Kriterium ist die Notwendigkeit, daß die freigabefördernden Substanzen in der Anwendungs-konzentration bei der Lagerung des Silicon-Kautschuks nicht ausschwitzen. Die Konzentration beträgt daher 2 bis 50 Gew.%, vorzugsweise 5 bis 40 Gew.%, bezogen auf Silicon-Kautschuk.

Geeignete, freigabefördernde Zusätze sind daher z.B. Alkohole, wie 2-Octyldodecanol, Oleylalkohol, Phenyläthanol, Ester, wie Myristinsäure-isopropylester, Caprylcaprinsäure-laurylstearylester, Laurinsäurehexylester (®Cetiol A), Propionsäure-myristylester, Isostearinsäure-äthyllauryl-ester, Ölsäure-äthylester, Essigsäurephenylester, Benzoesäure-benzylester, Salicylsäure-methylester, Laurinsäure-mono-1,2-propandiolester, Fettsäurepolyäthylenglykolester, Caprylcaprinsäure-1,2-propandiol-diester (®Miglyol 840), Caprylcaprinsäure-glycerinmonoester, Laurinsäure-glycerin-diester, Buttersäureglycerintriester, Caprylcaprinlaurinsäure-glycerintriester, Miglyol 812) Essigstearinölsäureglycerintriester, Adipinsäure-dibutylester, Sebacinsäure-dibutylester, Phthalsäureester, Citronensäure-triäthylester (®Citroflex 2), Äther, wie Didecyläther, Fettalkohol-polyäthylenglykoläther, Alkylaryl-polyäthylenglykoläther, Anisol und Ketone, wie Methylnonylketon.

Aus den genannten Gründen sind Zusätze von Fettsäureestern mittleren Molekulargewichts vom Typ des Myristinsäure-isopropylesters, des Laurinsäure-hexylesters oder des Caprylcaprinsäure-1,2-propandioldiesters sowie von Äthern mittleren Molekulargewichts vom Typ des Didecyläthers zu bevorzugen.

Die Zusätze von solchen die Wirkstoff-Freigabe fördernden Substanzen machen es möglich, Wirkstoffe, wie z.B. Anabolika, in wirksamen Dosen über längere Zeiträume mit Hilfe einer Nasenspange Tieren zuzuführen, ohne daß, falls die Tiere zum menschlichen Verzehr bestimmt sind, Rückstandprobleme auftreten.

Der Depotkörper kann ein oder mehrere Wirkstoffe enthalten. Der Wirkstoff wird von dem Silicon-Kautschuk ganz oder teilweise umhüllt. Er kann in gelöster oder dispergierter Form vorliegen oder aber auch in Form mehr oder weniger großer Wirkstoffteilchen oder wirkstoffhaltiger Teile wie z.B. Kristalle, Granulate, Pulver mit und ohne wieteren Zuschlagstoffen. Eine gleichmäßige Verteilung im Silicon-Kautschuk ist nicht erforderlich, vielmehr kann sich der in nicht gelöster oder dispergierter Form vorliegende Wirkstoff auch in einer oder mehreren Kammern des Depotkörpers befinden. Der Wirkstoff kann auch in einer oder mehreren Tablette(n), welche zusätzlich Polyäthylenglykol(e) und weitere Hilfstoffe enthalten kann (können), im Depotkörper vorliegen. Ein Depotkörper kann ein oder mehrere Tabletten enthalten. Bevorzugt ist ein solcher, der eine Tablette enthält, die nur teilweise vom Silicon-Kautschuk umhüllt ist.

Die erfindungsgemäßen Depotkörper auf Basis Silikon-Kautschuk werden vorzugsweise auf Schleimhäute insbesondere unter Anwendung einer Nasenspange appliziert. Sie haben den Vorteil, daß sie infolge ihrer Elastizität die Gesamt-Elastizität der zu verwendenden Vorrichtung erhöhen und damit besonders über lange Zeiträume bei Tieren die Tragbarkeit und Reizlosigkeit verbessern.

Solche Arzneiformen eignen sich zum Beispiel für die Applikation von Steroid-Hormonen. Sie können aber auch für andere Wirkstoffe, wie Antibiotika, Chemotherapeutika, Prostaglandine oder Vitamine eingesetzt werden.

Als Wirkstoffe zur Applikation auf die Schleimhaut des Nasenvorhofs der Rinder stehen Anabolika natürlicher, partialsynthetischer oder synthetischer Herkunft im Vordergrund des Interesses. Sie verbessern den Fleischansatz und damit die Schlachtkörperqualität. Solche Anabolika sind zum Beispiel Testosteron oder Trenbolon, gegebenenfalls in Kombination mit Östrogenen, wie Östradiol-17ß. Diese

**0013949**

Verbindungen können als Alkohole oder in Form ihrer Derivate, wie Ester oder Äther, angewendet werden (vgl. zum Beispiel DE—OS 2 323 615).

Die Depotkörper eignen sich auch zur prophylaktischen und therapeutischen Behandlung von Tieren.

Die erfindungsgemäßen Depotkörper können verschiedene Formen aufweisen, je nach Art ihrer Applikation. Sie können mehr oder minder abgerundet, würfel- oder quaderförmig, zylindrisch oblongförmig, oval oder sonst in einer Form ausgebildet sein, wobei die Oberfläche glatt oder strukturiert sein kann. Wenn sie im Nasenvorhof appliziert werden, sollen sie der Schleimhaut aufliegen und die Atemluft nicht behindern. Die in den Nasenvorhof einzusetzenden Depotkörper können identisch oder unterschiedlich sein.

Die Herstellung der Depotkörper ist dadurch gekennzeichnet, daß in der Silicon-Masse vor ihrer Vulkanisation die freigabefördernde(n) Substanz(en) gelöst, der Wirkstoff oder die Wirkstoffe umhüllt, gelöst oder dispergiert werden und daß schließlich vernetzt wird.

Die Zusammensetzung und Herstellung der Depotkörper wie auch die Prüfung und die Ergebnisse der Wirkstoffe-Freigabe in vitro wird in den Beispielen 1 bis 3 beschrieben. In Abhängigkeit von der Größe des Tieres bzw. seines Nasenvorhofs können die Abmessungen der Depotkörper und die Dosierung der Wirkstoffe von den hier angegebenen Werten abweichen, was auch eine Modifizierung der Methode zu Bestimmung der Wirkstoff-Freigabe zur Folge haben kann. Die Beispiele stellen nur eine Erläuterung der Erfindung dar.

Die Wirksamkeit der beanspruchten Depotkörper im Tierversuch ist dem Beispiel 4 zu entnehmen.

Beispiel 1

Herstellung von 16 Depotkörpern

a) - 10,712 g Dimethylpolysiloxan (additionsvernetzend) Komponente A wurden mit 2,976 g Capryl-caprinsäure-1,2-propandioldiester (entsprechend 20% freigabefördernder Substanz) gemischt.

Zu dieser Mischung wurden 1,192 g Dimethylpolysiloxan Komponente B gegeben. Unter Rühren wurde zum Zwecke des Entlüftens evakuiert. Nach Unterbrechen des Vakuums wurden 3,200 g mikrofeines Testosteron und 0,320 g mikrofeines Östradiol-17ß auf die Oberfläche des Silicon-Gemisches gegeben. Es wurde erneut evakuiert. Danach wurden die Feststoffe im Silicon-Gemisch durch Rühren dispergiert. Der beschriebene Vorgang lief bei Raumtemperatur innerhalb 10 Minuten ab. Die Rührgeschwindigkeit betrug maximal 250 U · Min.$^{-1}$. Danach wurde die Masse in Formen gegossen. Es wurde 30 Min. bei +70°C getempert und schließlich ausgeschaltet.

Der Depotkörper hatte zylindrische Form mit einem Durchmesser von 15 mm und einer Höhe von 8 mm, ein Gewicht von 1,15 g entsprechend einem Wirkstoffgehalt von 0,2 g Testosteron und 0,02 g Östradiol-17ß. Die Oberseite war kalottenförmig abgerundet. Die Unterseite wurde durch eine aus Kunststoff bestehende Platte mit Befestigungsvorrichtungen bedeckt.

Weitere Zusammensetzungen der Depotkörper sind den Tabellen 3.1—3.5 zu entnehmen.

Beispiel 2

Herstellung von 16 Depotkörpern

21 g Trenbolonacetat mikrofein, 4 g Östradiol-17ß (fr. Alk.) mikrofein und 26,7 g Polyäthylenglykol 4000 Typ Pulver wurden gemischt, vorgepreßt, durch ein Sieb von 1,5 mm granuliert und zu biplanen Tabletten von 11 mm Durchmesser verpreßt.

10,876 g Dimethylpolysiloxan (additionsvernetzend) Komponente A wurden mit 0,636 g Caprylcaprinlaurinsäureglycerintriester (entsprechend 5% freigabefördernde Substanz) gemischt. Zu dieser Mischung wurden 1,208 g Dimethylpolysiloxan Komponente B gegeben. Unter Rühren wurde zum Zwecke des Entlüftens bei Raumtemperatur 5 Min. evakuiert.

Eine Tablette wurde auf eine aus Kunststoff bestehende, mit Befestigungsvorrichtung versehene Platte gelegt und in einer geeigneten Form mit dem entlüfteten Silicon-Gemisch umgossen. Es wurde 60 min. bei +40°C getempert und danach ausgeschalt. Die Schichtdicke des Silicon-Kautschuks betrug 2 mm.

Der Depotkörper hatte zylindrische Form mit einem Durchmesser von 15 mm und einer Höhe von 8 mm, ein Gewicht von 1,0535 g entsprechend einem Wirkstoffgehalt von 0,125 g Trenbolonacetat und 0,02 g Östradiol-17ß. Die Oberseite war kalottenförmig abgerundet. Die Unterseite wurde durch eine aus Kunststoff bestehende Platte mit befestigungsvorrichtungen bedeckt.

Weitere Zusammensetzungen sind Tabelle 3.6 zu entnehmen.

Beispiel 3

Methode zur Prüfung der Wirkstoff-Freigabe in vitro von Depotkörpern mit einem Gehalt von 210 bis 280 mg Trenbolonacetat oder von 200 bis 400 mg Testosteron, gegebenenfalls in Kombination mit 40 bis 60 mg Östradiol-17ß bzw. Östradiol-diacetat.

Erlenmeyer-Kolben mit 300 ml Nennvolumen wurden mit 250 ml entsalztem Wasser gefüllt. Sie wurden mit Schliffstopfen verschlossen, die an ihrem dem Kolbeninnern zugewandten Teil mit einer Befestigung für die mit den Depotkörpern verbundenen Halterungen versehen waren. Die Befestigung war so lang, daß die Depotkörper in das Wasser eintauchten und 1 cm vom Kolbenboden entfernt

4

# 0013949

waren. Die Kolben wurden bei +38,5°C in einer Inkubationsschüttelmaschine (zum Beispiel Luftbad von Fa. Braun oder Wasserbad von Fa. Infors) mit einer Frequenz von 200/Min. bewegt.

Das zum eluieren benutzte Wasser wurde täglich ersetzt. Im Eluat wurde gegebenenfalls nach Verdünnen mit Wasser der Gehalt an Testosteron oder Trenbolonacetat direkt spektrophotometrisch bestimmt. Die Gehalte an Östradiol-17ß wurden nach Vereinigen der Eluate einer Woche und Anreicherung auf einer Ionenaustauschersäule kolorimetrisch mit Dansylchlorid bestimmt.

Die Ergebnisse der Wirkstoff-Freigabe aus erfindungsgemäßen Depotkörpern verschiedener Zusammensetzungen sind in den folgenden Tabellen enthalten. Zusätzlich wurde in diesen Tabellen auch die Freigabe aus einem Depotkörper ohne Zusatz einer freigabefördernden Substanz zum Vergleich angegeben.

## TABELLE 3.1

Vergleich der Testosteron-Freigabe in vitro aus Silicon-Kautschuk Typ Silgel 601 mit Zusatz von gelösten, schwer wasserlöslichen Stoffen. Die Angaben beziehen sich auf je zwei Depotkörper von gleichem Volumen und gleicher Oberfläche (siehe Beispiel 1).

### 3.1.1

| Zusammensetzung | mg | mg | mg | mg |
|---|---|---|---|---|
| Testosteron (fr.Alk.) mikrofein | 200 | 200 | 300 | 300 |
| Östradiol-17ß (fr. Alk.) mikrofein | 40 | 40 | 60 | 40 |
| 2-Octyl-dodecanol | — | 115 | — | — |
| Caprylcaprinlaurinsäure-glycerinester | — | — | — | 63 |
| Silgel 601 | 1950 | 1945 | 1240 | 1197 |
| Gesamtgewicht | 2190 | 2300 | 1600 | 1600 |

| Testosteron-Freigabe in vitro | mg | mg | mg | mg |
|---|---|---|---|---|
| in 1 Tag | 4 | 9 | 10 | 12 |
| in 4 Tagen | 10 | 19 | 20 | 29 |
| in 11 Tagen | 19 | 36 | 34 | 51 |
| in 14 Tagen | — | — | 40 | 57 |
| in 15 Tagen | — | — | 41 | 59 |

5

TABELLE 3.1.2.

| Zusammensetzung | mg | mg | mg | mg | mg | mg | mg | mg | mg[+] | mg | mg | mg | mg | mg |
|---|---|---|---|---|---|---|---|---|---|---|---|---|---|---|
| Testosteron (fr. Alk.) mikrofein | 400 | 400 | 400 | 400 | 400 | 400 | 400 | 400 | 400 | 400 | 400 | 400 | 400 | 469 |
| Östradiol-17ß (fr. Alk.) mikrofein | 40 | 40 | 40 | 80 | 40 | 40 | 40 | 40 | 40 | 80 | 40 | 40 | 80 | 38 |
| Myristinsäure-isopropylester | — | 93 | 372 | — | — | — | — | — | — | 182 | — | — | — | — |
| Laurinsäure-hexylester | — | — | — | 364 | — | — | — | — | — | — | — | — | — | — |
| Caprylcaprinsäurelauryl-stearyleaster | — | — | — | — | 93 | 372 | — | — | — | — | — | — | — | — |
| Caprylcaprinsäure-1,2-propandiolester | — | — | — | — | — | — | 186 | 279 | 332 | 182 | — | — | — | — |
| Adipinsäure-dibutylester | — | — | — | — | — | — | — | — | — | — | 93 | — | — | — |
| Sebacinsäure-dibutylester | — | — | — | — | — | — | — | — | — | — | — | 93 | — | — |
| Phthalsäuredioctylester | — | — | — | — | — | — | — | — | — | — | — | — | 91 | — |
| Didecylether | — | — | — | — | — | — | — | — | — | — | — | — | — | 436 |
| Silgel 601 | 1860 | 1767 | 1488 | 1456 | 1767 | 1488 | 1674 | 1581 | 1328 | 1456 | 1767 | 1767 | 1729 | 1307 |
| Gesamtgewicht | 2300 | 2300 | 2300 | 2300 | 2300 | 2300 | 2300 | 2300 | 2100 | 2300 | 2300 | 2300 | 2300 | 2250 |

[+]="G"

3.1.2

| Testosteron-Freigabe in vitro | mg | mg | mg | mg | mg | mg | mg | mg | mg | mg | mg | mg | mg | mg |
|---|---|---|---|---|---|---|---|---|---|---|---|---|---|---|
| in 1 Tag | 13 | 16 | 32 | 21 | 14 | 28 | 24 | 21 | 20 | 20 | 19 | 16 | 14 | 28 |
| in 4 Tagen | 28 | 35 | 79 | 58 | 29 | 70 | 52 | 48 | 74 | 62 | 45 | 41 | 28 | 69 |
| in 11 Tagen | 48 | 56 | 137 | 101 | 47 | 112 | 79 | 83 | 135 | 109 | 69 | 73 | 50 | 118 |
| in 14 Tagen | 52 | 61 | 152 | 114 | 52 | 124 | 87 | 92 | 151 | 123 | 76 | 83 | 53 | 133 |
| in 15 Tagen | 54 | 63 | 157 | — | 55 | 129 | 90 | 102 | 168 | — | 78 | 86 | 55 | 138 |
| in 21 Tagen | 65 | 68 | 184 | — | 60 | 152 | 106 | 120 | 187 | — | 89 | 102 | 67 | 164 |

**0 013 949**

TABELLE 3.2.

Vergleich der Testosteron-Freigabe in vitro aus Silicon-Kautschuk auf Basis eines Additionsvernetzenden Zweikomponentensystems auf Basis Dimethyldiphenylpolysiloxan (nachfolgend als Silicon SLM 71260 bezeichnet) mit Zusatz von gelösten, schwer wasserlöslichen Stoffen. Die Angaben beziehen sich auf je zwei Depotkörper von gleichem Volumen und gleicher Oberfläche (siehe Beispiel 1).

3.2.1.

| Zusammensetzung | mg | mg | mg | mg |
|---|---|---|---|---|
| Testosteron (fr. Alk.) mikrofein | 300 | 300 | 300 | 300 |
| Caprylcaprinsäure-glycerinester | — | 260 | — | — |
| Caprylcaprinlaurinsäure-glycerinester | — | — | 260 | — |
| Caprylcaprinsäure-1,2-propandiolester | — | — | — | 260 |
| Silicon SLM 71260 | 1300 | 1040 | 1040 | 1040 |
| Gesamtgewicht | 1600 | 1600 | 1600 | 1600 |

| Wifstoff-Freigabe in vitro | mg | mg | mg | mg |
|---|---|---|---|---|
| in 1 Tag | 20 | 22 | 20 | 22 |
| in 4 Tagen | 45 | 76 | 71 | 81 |
| in 14 Tagen | 81 | 158 | 145 | 154 |

3.2.2.

| Zusammensetzung | mg | mg |
|---|---|---|
| Testosteron (fr. Alk.) mikrofein | 300 | 400 |
| Caprylcaprinsäure-1,2-propandiolester | 260 | 240 |
| Silicon SLM 71260 | 1040 | 960 |
| Gesamtgewichte | 1600 | 1600 |

| Wirkstoff-Freigabe in vitro | mg | mg |
|---|---|---|
| in 1 Tag | 22 | 31 |
| in 4 Tagen | 81 | 93 |
| in 14 Tagen | 154 | 178 |
| in 21 Tagen | 180 | 207 |

3.2.3.

| Zusammensetzung | mg | mg | mg | mg | mg | mg | mg[+] | mg |
|---|---|---|---|---|---|---|---|---|
| Testosteron (fr. Alk.) mikrofein | 400 | 400 | 400 | 400 | 400 | 400 | 400 | 400 |
| Östradiol-17ß (fr. Alk.) mikrofein | — | 40 | — | 40 | — | — | 40 | 80 |
| Östradiol-17ß-diacetat mikrofein | 52 | — | 52 | — | 52 | 52 | — | — |
| Caprylcaprinsäure-1,2-propandiolester | — | — | 369 | 372 | 554 | 739 | 664 | — |
| Laurinsäure-hexylester | — | — | — | — | — | — | — | 364 |
| Silicon SLM 71260 | 1848 | 1860 | 1479 | 1488 | 1294 | 1109 | 996 | 1456 |
| Gesamtgewicht | 2300 | 2300 | 2300 | 2300 | 2300 | 2300 | 2100 | 2300 |

[+] = "F"

7

| Testosteron-Freigabe in vitro | mg | mg | mg | mg | mg | mg | mg | mg |
|---|---|---|---|---|---|---|---|---|
| in 1 Tag | 15 | 15 | 22 | 22 | 21 | 21 | 23 | 21 |
| in 4 Tagen | 36 | 41 | 76 | 80 | 79 | 83 | 80 | 70 |
| in 14 Tagen | 68 | 75 | 142 | 142 | 146 | 173 | 177 | 138 |
| in 16 Tagen | 74 | 81 | 152 | 153 | 159 | 191 | 202 | 150 |
| in 28 Tagen | — | 117 | — | — | 207 | 257 | 285 | — |
| in 43 Tagen | — | 141 | — | — | 254 | 315 | 357 | — |

3.3.
Vergleich der Trenbolonacetat-Freigabe in vitro aus Silicon-Kautschuk Typ Silgel 601 mit Zusatz von gelösten, schwer wasserlöslichen Stoffen. Die Angaben beziehen sich auf zwei Depotkörper von gleichem Volumen und gleicher Oberfläche (siehe Beispiel 1).

| Zusammensetzung | mg | mg | mg | mg | mg | mg | mg |
|---|---|---|---|---|---|---|---|
| Trenbolonacetat mikrofein | 210 | 210 | 210 | 210 | 210 | 210 | 210 |
| Östradiol-17ß (fr. Alk.) mikrof. | 40 | 40 | 40 | 40 | 40 | 40 | 40 |
| Myristinsäure-isopropylester | — | 103 | 410 | — | — | — | — |
| Caprylcaprinsäure-laurylstearylester | — | — | — | 103 | — | — | — |
| Caprylcaprinlaurinsäure-glycerinester | — | — | — | — | 103 | — | — |
| Caprylcaprinsäure-1,2-propandiolester | — | — | — | — | — | 410 | — |
| Adipinsäure-dibutylester | — | — | — | — | — | — | 103 |
| Silgel 601 | 2050 | 1947 | 1640 | 1947 | 1947 | 1640 | 1947 |
| Gesamtgewicht | 2300 | 2300 | 2300 | 2300 | 2300 | 2300 | 2300 |

| Trenbolonacetat-Freigabe in vitro | mg | mg | mg | mg | mg | mg | mg |
|---|---|---|---|---|---|---|---|
| in 1 Tag | 15 | 10 | 13 | 11 | 20 | 20 | 17 |
| in 4 Tagen | 48 | 50 | 63 | 47 | 60 | 70 | 59 |
| in 14 Tagen | 109 | 115 | 153 | 113 | 123 | 158 | 126 |
| in 21 Tagen | 139 | 137 | 184 | 133 | — | 174 | — |
| in 28 Tagen | 158 | — | — | — | — | — | — |
| in 42 Tagen | 189 | — | — | — | — | — | — |

3.4.1.
Vergleich der Östradiol-17ß-Freigabe in vitro aus Silicon-Kautschuk Typ Silgel 601 mit Zusatz von gelösten, schwer wasserlöslichen Stoffen. Die Angaben beziehen sich auf je zwei Depotkörper von gleichem Volumen und gleicher Oberfläche (siehe Beispiel 1).

| Zusammensetzung | mg | mg | mg | mg | mg[+] | mg | mg | mg |
|---|---|---|---|---|---|---|---|---|
| Testosteron (fr. Alk.) mikrofein | 400 | 400 | 400 | 400 | 400 | 400 | 400 | 469 |
| Ostradiol-17ß (fr. Alk.) mikrofein | 40 | 40 | 80 | 40 | 40 | 40 | 40 | 38 |
| Myristinsäure-isopropylester | — | 372 | — | — | — | — | — | — |
| Laurinsäure-hexylester | — | — | 364 | — | — | — | — | — |
| Caprylcaprinsäure-laurylstearylester | — | — | — | 372 | — | — | — | — |
| Caprylcaprinsäure-1,2-propandiolester | — | — | — | — | 332 | — | — | — |
| Adipinsäure-dibutylester | — | — | — | — | — | 93 | — | — |
| Sebacinsäure-dibutylester | — | — | — | — | — | — | 93 | — |
| Didecylether | — | — | — | — | — | — | — | 436 |
| Silgel 601 | 1860 | 1488 | 1456 | 1488 | 1328 | 1767 | 1767 | 1307 |
| Gesamtgewicht | 2300 | 2300 | 2300 | 2300 | 2100 | 2300 | 2300 | 2250 |

[+] = "G"

| Östradiol 17ß-Freigabe in vitro | mg | mg | mg | mg | mg | mg | mg | mg |
|---|---|---|---|---|---|---|---|---|
| in 1 Woche | 1,10 | 4,62 | 5,72 | 4,26 | 5,64 | 2,62 | 2,50 | 3,72 |
| in 2 Wochen | 1,72 | 8,16 | — | 7,88 | 10,30 | 3,56 | 3,78 | 5,74 |
| in 3 Wochen | 2,26 | 10,34 | — | 10,82 | 13,80 | 4,18 | 4,96 | 7,82 |
| in 4 Wochen | 3,02 | 12,64 | — | — | 16,56 | — | — | 9,62 |
| in 5 Wochen | 3,36 | — | — | — | 19,04 | — | — | 10,96 |
| in 6 Wochen | 4,34 | — | — | — | 20,94 | — | — | 12,46 |

3.4.2.

Vergleich der Östradiol-17ß-Freigabe in vitro aus Silicon-Kautschuk Typ SLM 71 260 mit Zusatz von gelösten, schwer wasserlöslichen Stoffen. Die Angaben beziehen sich auf je zwei Depotkörper von gleichem Volumen und gleicher Oberfläche (siehe Beispiel 1).

| Zusammensetzung | mg | mg | mg | mg | mg | mg[+] |
|---|---|---|---|---|---|---|
| Testosteron (fr. Alk.) mikrofein | 400 | 400 | 400 | 400 | 400 | 400 |
| Östradiol-17ß mikrofein | 40 | 80 | 40 | 40 | 40 | 40 |
| Laurinsäure-hexylester | — | 364 | — | — | — | — |
| Essigstearinölsäure-glycerinester | — | — | — | 93 | — | — |
| Caprylcaprinsäure-1,2-propandiolester | — | — | 93 | — | 372 | 664 |
| Silicon SLM 71260 | 1860 | 1456 | 1767 | 1767 | 1488 | 996 |
| Gesamtgewicht | 2300 | 2300 | 2300 | 2300 | 2300 | 2100 |

[+] = "F"

| Östradiol-17ß-Freigabe in vitro | mg | mg | mg | mg | mg | mg |
|---|---|---|---|---|---|---|
| in 1 Woche | 3,10 | 6,52 | 3,96 | 4,68 | 6,26 | 6,48 |
| in 2 Wochen | 4,12 | 11,68 | 6,24 | 7,96 | 11,28 | 13,76 |
| in 3 Wochen | 5,58 | 15,86 | 8,34 | 9,84 | — | 19,54 |
| in 4 Wochen | 7,74 | — | — | — | — | 24,56 |
| in 5 Wochen | 8,62 | — | — | — | — | 29,08 |
| in 6 Wochen | 10,16 | — | — | — | — | 32,84 |

3.5.

Vergleich der Ostradiol-17ß-Freigabe in vitro aus Silicon-Kautschuk Typ Silgel 601 mit Zusatz von gelösten schwer wasserlöslichen Stoffen. Die Angaben beziehen sich auf je zwei Depotkörper von gleichem Volumen und gleicher Oberfläche (siehe Beispiel 1).

| Zusammensetzung | mg | mg | mg | mg | mg | mg |
|---|---|---|---|---|---|---|
| Trenbolonacet mikrofein | 210 | 210 | 210 | 210 | 210 | 210 |
| Östradiol-17ß mikrofein | 40 | 40 | 40 | 40 | — | — |
| Ostradiol-17ß-diacetate mikrofein | — | — | — | — | 52 | 52 |
| Caprylcaprinlaurinsäure-glycerinester | — | 103 | — | — | — | 102 |
| Caprylcaprinsäure-1,2-propandiolester | — | — | 410 | — | — | — |
| Myristinsäure-isopropylester | — | — | — | 410 | — | — |
| Silgel 601 | 2050 | 1947 | 1640 | 1640 | 2038 | 1936 |
| Gesamtgewicht | 2300 | 2300 | 2300 | 2300 | 2300 | 2300 |

| Wirkstoff-Freigabe in vitro | mg | mg | mg | mg | mg | mg |
|---|---|---|---|---|---|---|
| in 1 Woche | 0,44 | 1,96 | 2,18 | 2,44 | 1,36 | 1,90 |
| in 2 Wochen | 1,08 | 3,32 | 4,42 | 4,32 | 2,66 | 3,26 |
| in 3 Wochen | 1,76 | — | 6,30 | 6,9 | 4,28 | — |
| in 4 Wochen | 2,30 | — | — | — | 5,76 | — |
| in 5 Wochen | 2,70 | — | — | — | 7,18 | — |
| in 6 Wochen | 3,08 | — | — | — | 8,68 | — |

# 0013949

## 3.6.

Vergleich der Trenbolonacetat-Freigabe in vitro durch eine 2 mm dicke Schicht aus Silicon-Kautschuk Typ Silgel 601 bzw. Silicon SLM 71260 mit Zusatz von gelösten, schwer wasserlöslichen Stoffen. Die Angaben der Tabelle beziehen sich auf zwei Depotkörper gleicher Art (siehe Beispiel 2).

| Zusammensetzung | mg | mg | mg | mg |
|---|---|---|---|---|
| Tablette | | | | |
| Trenbolonacetat mikrofein | 210 | 210 | 210 | 210 |
| Östradiol-17ß (fr. Alk.) mikrofein | 40 | 40 | 40 | — |
| Östradiol-17ß-diacetat mikrofein | — | — | — | 52 |
| Polyäthylenglykol 4000 | 267 | 267 | — | — |
| Polyäthylenglykol 6000 | — | — | 267 | 255 |
| Silicon-Schicht | | | | |
| Caprylcaprinlaurinsäure-glycerinester | — | 79 | — | — |
| Caprylcaprinsäure-1,2-propandiolester | — | — | — | 636 |
| Silgel 601 | 1590 | 1511 | — | — |
| Silicon SLM 71260 | — | — | 1590 | 954 |

| Trenbolonacetat-Freigabe in vitro | mg | mg | mg | mg |
|---|---|---|---|---|
| in 1 Tag | 4 | 4 | 4 | 13 |
| in 4 Tagen | 12 | 14 | 17 | 57 |
| in 14 Tagen | 42 | 49 | 51 | 138 |
| in 21 Tagen | 59 | 77 | 75 | 193 |
| in 28 Tagen | 84 | 103 | 107 | 236 |
| in 35 Tagen | 105 | 125 | — | — |
| in 42 Tagen | 130 | 152 | — | — |

Beispiel 4

- Wirksamkeitsversuch an Milchmastkälbern mit Depotkörpern, wie in den Tabellen 3.1.2."G" bzw. 3.2.3."F" für Testosteron und 3.4.1."G" bzw. 3.4.2."F" füs Östradiol-17ß beschrieben.

Versuchsdurchführung

2 Gruppen von je 7 Milchmastkälbern mit einem Durchschnittsgewicht von 75,4 bzw. 74,8 kg wurden Nasenspangen entsprechend DB 2 125 464 mit Depotkörpern eingesetzt und für 6 Wochen belassen. Eine 3. Gruppe von 6 Kälbern mit einem Durchschnittsgewicht von 76,5 kg blieb unbehandelt und diente als Kontrolle.

Zur Feststellung der Gewichtsentwicklung wurden die Kälber dreimal im Abstand von 14 Tagen gewogen und die tägliche Gewichtszunahme sowie die Futterverwertung (Futterverbrauch: Gewichtszunahme) pro Gruppe während der Behandlungsperiode errechnet.

Ergebnisse

In den nachfolgenden Tabellen sind die Ergebnisse der täglichen Gewichtszunahme, der Futterverwertung sowie die kumulative Gewichtsentwicklung der Gruppen 1—3 (3=Kontrollgruppe) zusammengestellt.

## 0013949

### Mittelwerte der täglichen Gewichtsveränderungen in g und %

| Gruppe | Zeit | | | | | |
|---|---|---|---|---|---|---|
| | 1, und 2. Woche | | 3. und 4. Woche | | 5. und 6. Woche | |
| 1   n=7 entspr. "G" | 1377 | 126% | 1337 | 153% | 1133 | 129% |
| 2   n=7 entspr. "F" | 1367 | 125% | 1327 | 152% | 1255 | 143% |
| 3   n=6 Kontrolle | 1095 | 100% | 869 | 100% | 881 | 100% |

### Gruppenmittel der Futterverwertung während der 6wöchigen Versuchsperioden

| Gruppe 1   n=7 entspr. "G" | Gruppe 2   n=7 entspr. "F" | Gruppe 3   n=6 Kontrolle |
|---|---|---|
| 1,45 +19,9% | 1,44 +20,6% | 1,81 ±0 |

### Kumulative Gewichtsentwicklung in kg und %

| Durchschnitt d. Gewichtszunahme im Zeitraum | Gruppe 1   n=7 entspr. "G" | | Gruppe 2   n=7 entspr. "F" | | Gruppe 3   n=6 Kontrolle | |
|---|---|---|---|---|---|---|
| bis   2. Woche | 19,3 | 126,1% | 19,1 | 124,8% | 15,3 | 100% |
| bis   4. Woche | 38,0 | 138,2% | 37,7 | 137,1% | 27,5 | 100% |
| bis   6. Woche | 53,9 | 135,4% | 55,3 | 138,9% | 39,8 | 100% |

Die Befunde zeigen, daß mit den Depotkörpern entsprechend "G" sowie "F", verabreicht mittels Nasenspange, gegenüber einer Kontrolle (Gruppe 3) Mehrzunahmen von 14,1 bzw. 15,5 kg/Kalb erreicht werden. Hinzu kommt eine um ca. 20% verbesserte Futterverwertung im Vergleich zur Kontrolle.

Die Einzelergebnisse der kumulativen Gewichtsentwicklung ließen sich mit paarweisem Vergleich im T-Test nach Student (P<0,01) statistisch sichern.

**Patentansprüche**

1. Depotkörper auf Basis Silicon-Kautschuk zur protrahierten Wirkstoff-Freigabe, gegebenenfalls mit festen Zuschlagstoffen, dadurch gekennzeichnet, daß der Depotkörper 2 bis 50 Gew.%, bezogen auf Silicon-Kautschuk, einer Freigabefördernden Substanz ausgewählt aus der Gruppe bestehend aus Alkohol, Ester, Äther und Keton mit einer Anzahl von 8 bis 60 C-Atomen, einzeln oder im Gemisch in gelöster Form enthält.

2. Depotkörper gemäß Anspruch 1, dadurch gekennzeichnet, daß er als freigabefördernde Substanzen Myristinsäureisopropylester, Laurinsäure-hexylester, Caprylcaprinsäure-1,2-propandioldiester oder Didecyläther enthält.

3. Depotkörper gemäß Anspruch 1, dadurch gekennzeichnet, daß die freigabefördende(n) Substanz(en) in der Konzentration von 5—40 Gew.% enthalten ist/sind.

4. Depotkörper gemäß Anspruch 1, dadurch gekennzeichnet, daß er Silicon-Kautschuk auf Basis von durch Addition oder Kondensation vernetzten Ein- oder Zweikomponenten-Systemen enthält.

5. Depotkörper gemäß Ansprüchen 1 und 4, dadurch gekennzeichnet, daß er Silicon-Kautschuk auf Basis Dimethylpolysiloxan, Dimethyldiphenylpolysiloxan, Dimethylpolysiloxanol oder Silicon-Copolymeren enthält.

6. Depotkörper gemäß Anspruch 1, dadurch gekennzeichnet, daß er einen oder mehrere Wirkstoff(e) in gelöster oder dispergierter Form enthält und daß der Silicon-Kautschuk ihn oder sie teilweise oder ganz umhüllt.

11

7. Depotkörper gemäß Anspruch 1, dadurch gekennzeichnet, daß er einen oder mehrere Wirkstoff(e) in einer oder mehreren Kammern enthält.

8. Depotkörper gemäß Anspruch 1, dadurch gekennzeichnet, daß er einen oder mehrere Wirkstoff(e) und gegebenenfalls Polyäthylenglykol(e) und weitere Hilfsstoffe in einer oder mehreren Tablette(n) enthält.

9. Depotkörper gemäß Anspruch 1, dadurch gekennzeichnet, daß er als Wirkstoff(e) Steroide, gegebenenfalls als Kombination von Androgenen und Östrogenen, Antibiotika, Chemotherapeutika, Prostaglandine oder Vitamine enthält.

10. Depotkörper gemäß Ansprüchen 1 und 9, dadurch gekennzeichnet, daß er als Wirkstoff(e) Testosteron und seine Ester, Trenbolon und seine Ester, gegebenenfalls in Kombination mit Östradiol-17ß und seinen Estern, enthält.

11. Depotkörper gemäß Anspruch 1, dadurch gekennzeichnet, daß er zur Applikation auf die Schleimhaut des Nasenvorhofes von Rindern geeignet ist.

12. Depotkörper gemäß Anspruch 1, dadurch gekennzeichnet, daß er zur Verbindung mit Vorrichtungen geeignet ist, die die Fixation der Depotkörper auf der Schleimhaut im Nasenvorhof von Rindern gewährleisten.

13. Verfahren zur Herstellung der Depotkörper gemäß Anspruch 1, dadurch gekennzeichnet, daß in der Silicon-Kautschuk-Masse vor ihrer Vulkanisation 2 bis 50 Gewichtsprozent bezogen auf Silicon-Kautschuk einer freigabefördernden Substanz oder eines freigabefördernden Substanzgemisches gelöst, der Wirkstoff oder die Wirkstoffe umhüllt, gelöst oder dispergiert werden und daß schleißlich vulkanisiert wird.

**Revendications**

1. Corps de dépôt à base de caoutchouc de silicone pour une libération retardée de la substance active, le cas échéant avec des additifs solides, caractérisé en ce que le corps de dépôt contient sous forme dissoute 2 à 50% en poids, par rapport au caoutchouc de silicone, d'une substance favorisant la libération, choisie parmi le groupe formé par les alcools, esters, éthers, et cétones ayant de 8 à 60 atomes de carbone, seuls ou en mélange.

2. Corps de dépôt suivant la revendication 1, caractérisé en ce qu'il contient comme substances favorisant la libération du myristate d'isopropyle, du laurate d'hexyle, du caprylcaprate de 1,2-propanediol ou de l'éther didécylique.

3. Corps de dépôt suivant la revendication 1, caractérisé en ce que la ou les substance(s) favorisant la libération est/sont contenue(s) à une concentration de 5 à 40% en poids.

4. Corps de dépôt suivant la revendication 1, caractérisé en ce qu'il contient un caoutchouc de silicone à base de systèmes à un ou deux constituants réticulés par addition ou condensation.

5. Corps de dépôt suivant les revendications 1 et 4, caractérisé en ce qu'il contient un caoutchouc de silicone à base de polydiméthylsiloxane, de polydiméthyldiphénylsiloxane, de polydiméthylsiloxanol ou de copolymères de silicones.

6. Corps de dépôt suivant la revendication 1, caractérisé en ce qu'il contient une ou plusieurs substance(s) active(s) sous forme dissoute ou dispersée et en ce que le caoutchouc de silicone l'enrobe ou les enrobe partiellement ou totalement.

7. Corps de dépôt suivant la revendication 1, caractérisé en ce qu'il contient une ou plusieurs substance(s) active(s) dans une ou plusieurs chambre(s).

8. Corps de dépôt suivant la revendication 1, caractérisé en ce qu'il contient une ou plusieurs substance(s) active(s) et le cas échéant du ou des polyéthylèneglycol(s) et d'autres adjuvants dans un ou plusieurs comprimé(s).

9. Corps de dépôt suivant la revendication 1, caractérisé en ce qu'il contient comme substance(s) active(s) des stéroïdes, le cas échéant sous forme de combinaison avec des androgènes et des oestrogènes, des antibiotiques, des médicaments chimiothérapeutiques, des prostaglandines ou des vitamines.

10. Corps de dépôt suivant les revendications 1 et 9, caractérisé en ce qu'il contient comme substance(s) active(s) de la testotérone et ses esters, la trenbolone et ses esters, le cas échéant en combinaison avec l'oestradiol-17ß et ses esters.

11. Corps de dépôt suivant la revendication 1, caractérisé en ce qu'il convient pour l'application sur la muqueuse du vestibule nasal des bovins.

12. Corps de dépôt suivant la revendication 1, caractérisé en ce qu'il convient pour la liaison avec des dispositifs qui assurent la fixation des corps de dépôt sur la muqueuse dans le vestibule nasal de bovins.

13. Procédé de préparation des corps de dépôt suivant la revendication 1, caractérisé en ce qu'on dissout dans la masse de caoutchouc de silicone avant la vulcanisation, 2 à 50% en poids par rapport au caoutchouc de silicone d'une substance favorisant la libération ou d'un mélange de substances favorisant la libération, en ce qu'on enrobe, dissout ou disperse la substance active ou les substances actives et en ce que finalement on vulcanise.

**0 013 949**

**Claims**

1. Depot body on the basis of silicone rubber for protraced release of active ingredient, optionally containing solid additives, wherein the depot body contains from 2 to 50 weight %, relative to silicone rubber, of a release-promoting substance, selected from the group consisting of alcohol, ester, ether and ketone having from 8 to 60 carbon atoms, *per se* or in a mixture thereof in dissolved form.

2. Depot body as claimed in claim 1, which comprises as release-promoting substances myristic acid isopropyl ester, lauric acid hexyl ester, caprylic/capric acid-1,2-propandiol diester or didecyl ether.

3. Depot body as claimed in claim 1, wherein the release-promoting substance(s) is/are contained in a concentration of from 5 to 40 weight %.

4. Depot body as claimed in claim 1, which comprises silicone rubber on the basis of single-component or two-component systems cross-linked by addition or condensation.

5. Depot body as claimed in claims 1 to 4, which comprises silicone rubber on the basis of dimethylpolysiloxane, dimethyl-diphenylpolysiloxane, dimethylpolysiloxanol or silicone copolymers.

6. Depot body as claimed in claim 1, which comprises one or more active substance(s) in dissolved or dispersed form which is/are wrapped partially or entirely by the silicone rubber.

7. Depot body as claimed in claim 1, which comprises one or more active substance(s) in one or more chambers.

8. Depot body as claimed in claim 1, which comprises one or more active substance(s) and optionally polyethyleneglycol(s) and further auxiliaries in one or more tablet(s).

9. Depot body as claimed in claim 1, which comprises as active substance(s) steroids, optionally as combination of androgens and estrogens, antibiotics, chemotherapeutical agents, prostaglandins or vitamins.

10. Depot body as claimed in claims 1 and 9, which comprises as active substance(s) testosterone and its esters, trenbolone and its esters, optionally in combination with estradiol-17ß and its esters.

11. Depot body as claimed in claim 1, which is suitable for application to the mucous membrane of the nasal vestibule of cattle.

12. Depot body as claimed in claim 1, which is suitable for connection with devices which ensure the fixing of the depot bodies on the mucous membrane of the nasal vestibule of cattle.

13. A process for the preparation ok the depot bodies as claimed in claim 1, which comprises dissolving in the silicone rubber mass before vulcanization from 2 to 50 weight %, relative to silicone rubber, of a release-promoting substance or a release-promoting substance mixture, wrapping or dissolving or dispersing the active ingredient or ingredients and subsequently vulcanizing.